# EUROPEAN PATENT SPECIFICATION

(11) **EP 1 290 161 B1**
(45) Date of publication and mention of the grant of the patent: **22.06.2011**
(21) Application number: 01935806.8
(22) Date of filing: 29.05.2001
(51) Int. Cl.: C12N 15/11, C12N 15/63

(54) **METHODS FOR MEDIATING GENE SUPPRESION BY USING FACTORS THAT ENHANCE RNAi**
METHODEN ZUR GENSUPPRESSION MITHILFE VON RNAi VERSTÄRKENDEN FAKTOREN
METHODES PERMETTANT D'AGIR SUR LA SUPPRESSION DE GENE PAR UTILISATION DE FACTEURS RENFOR ANT L'ARNI

(30) Priority: 30.05.2000 AU PQ783000; 07.08.2000 AU PQ924600
(43) Date of publication of application: 12.03.2003
(73) Proprietor: JOHNSON & JOHNSON RESEARCH PTY LIMITED, Eveleigh, NSW 1430 (AU)
(72) Inventor: ARNDT, Gregory, Martin, Malabar, NSW 2036 (AU); RAPONI, Mitch, Potts Point, NSW 2011 (AU)
(74) Representative: Wakerley, Helen Rachael
(86) International application number: PCT/AU2001/000627
(87) International publication number: WO 2001/092513

(56) References cited:
- WO-A-01/23604
- WO-A-99/32619
- MIYAKE S. & YAMASHITA S.: 'Identification of sna41 gene, which is the suppressor of nda4-mutation and is involved in DNA replication in schizosaccharomyces pombe' GENES TO CELLS vol. 3, no. 3, 1998, pages 157 - 166, XP002962428 & DATABASE GENBANK [Online] Database accession no. AB001739
- A. MARCHFELDER ET AL.: 'The gene for tibosomal protein L7a-1 in schizosaccharomyces pombe contains an intron after the initiation codon' BIOCHIMICA ET BIOPHYSICA ACTA vol. 1397, no. 2, 1998, pages 146 - 150, XP002962429 & DATABASE GENBANK [Online] Database accession no. AJ001133
- S. YOSHIOKA ET AL.: 'Identification of open reading frames in schizosaccharomyces pombe cDNAs' DNA RESEARCH vol. 4, 1997, pages 363 - 369, XP002917396 & DATABASE EMBL [Online] Database accession no. D89239
- R.F. KETTING ET AL.: 'mut-7 of C. elegans, required for transposon silencing and RNA interference, is a homolog of werner syndrome helicase and RNaseD' CELL vol. 99, 1999, pages 133 - 141, XP002962430
- J.M. BOSHER & M. LABOUESSE: 'RNA interference: genetic wand and genetic watchdog' NATURE CELL BIOLOGY vol. 2, February 2000, pages E31 - E36, XP001106960
- S. LIN-CHAO & S.N. COHEN: 'The rate of processing and degradation of antisense RNAi regulates the replication of Co1E1-type plasmids in vivo' CELL vol. 65, 1991, pages 1233 - 1242, XP002962431
- ARNDT G.M. ET AL.: 'Gene regulation by antisense RNA in the fission yeast schizosaccharomyces pombe' MOLECULAR AND GENERAL GENETICS vol. 248, no. 3, 1995, pages 293 - 300, XP002962432
- ATKINS D. ET AL.: 'The ade6 gene of the fission yeast as a target for antisense ribozyme RNA-mediates suppression' ANTISENSE RESEARCH AND DEVELOPMENT vol. 5, 1995, pages 295 - 305, XP002962433

### SEQUENCE LISTING

<110> JOHNSON & JOHNSON RESEARCH PTY LIMITED
<120> METHODS FOR MEDIATING GENE SUPPRESION
<130> 27329WOP00
<140> PQ7830 AND PQ9246
   <141> 2001-05-29
<160> 4
<170> PatentIn version 3.0
<210> 1
   <211> 1239
   <212> DNA
   <213> fission yeast
<400> 1
   tttaacttag ttcgctttgt taaattggcc tcgaggtcga cgttaattaa gccgcaattg     60
   taacagtaga ttttttgcat cattattact ctccgaaaca tgactgaaca ctcatttaag     120
   caaatagacg tgttttctaa taaaggtttt cgaggtaatc ctgttgcagt tttttttgat     180
   gcagataatt tatcacaaaa ggaaatgcag cagattgcca agtggacaaa tttatctgag    240
   acaacatttg ttcaaaagcc gacaatcgat aaagcagatt acagacttcg tatatttacc    300
   ccagaatgtg aattaagctt tgctggtcac ccaacaattg gatcgtgctt tgctgttgtt    360
   gaaagtggat attgtactcc aaaaaactgt aaaattattc aggaatgttt agccggttta    420
   gttgaattaa ctatcgatgg ggaaaaggat gaagacactt ggatttcttt caaacttccg    480
   tattacaaaa ttttacagac ttctgaaact gcaatttcag aagtagaaaa tgcattgggt    540
   attcctctga attatagttc tcaagtttct cctcctgtgt taatagatga tggaccaaag    600
   tggcttgtaa ttcaacttcc aaacgctaca gatgtgctca acctcgttcc gaaatttcag    660
   tccctttccc aagtttgtaa aaacaatgat tggataggcg tcacccgtct ttggtgaatt    720
   agaaaagact cgtttgaaag cccgaagctt tgcgccttta atacatgtca atgaggatcc    780
   ggcttgcggt agtggtgcag gagctgtcgg tgtgtatatt ggaagctctc aaaaaactcc    840
   aacttctcta tcatttacga tttctcaagg tacaaaatta agtagacaag caatttccaa    900
   agtcagcgta gacgtttcct ccaataaatc aattgctgtt tttgtcggtg gacaggcaaa    960
   aacttgtatt tctggaaaat cgtttattta atgtttttat tacaaatatt cacttgcgag   1020
   tttattttcc aatactgaag actttcaatc aatagcaaat atgctactca aggaagttca   1080
   ctcattcaaa agcaattggt ttactatatc gttttttcta actagttact agtcattgaa   1140
   caatctaccg aatgataaaa tgaaattttg gtttttcccc gggtaaaagg aatgtctccc   1200
   ttgccagtac tgctagggtt tttctttcga actataaga                          1239
<210> 2
   <211> 1105
   <212> DNA
   <213> fission yeast
<400> 2
   agtccgcttt gttaaattgg cctcgaggtc gacgttaatt aagcctgata tgatcgagct     60
   tgtcgaaatg gaaatgcgtg agctactctc cgaatacgga tttgatggtg acaatactcc    120
   aattgttagc ggcagtgctt tatgtgcctt agagggtcgt gagcctgaga ttggtctcaa    180
   tagtattact aaattgatgg aagctgttga tagttatatt actcttcctg aaagaaaaac    240
   ggatgtccct ttcttgatgg ccatcgagga cgttttttca atttcaggtc gcggaactgt    300
   agtcactggc cgtgtcgagc gcggtacttt aaagaagggt gctgaaatcg aaatcgtcgg    360
   ttatggtagc catttaaaga ctaccgttac tggaattgaa atgttcaaaa agcagcttga    420
   tgccgccgtt gccggtgaca attgtggcct tttacttcgt tctatcaagc gagagcaatt    480
   aaaacgtgga atgattgtcg ctcaaccagg aaccgttgct cctcatcaga aattcaaggc    540
   atcattctat attttgacaa aagaggaagg aggtcgtcgt acccggtttc gttgacaagt    600
   atcgtcccca actgtacagt ccgtacttcc gacgttactg tcgaacttac ccaccctgat    660
   cctaacgact caacaaaatg gttatgcctg gagacaatgt cgagatgatc tgtacgctta    720
   ttcaccccat tgtcatcgaa aaaggacaac gcttcacagt tcgtgagggt ggaagcactg    780
   taggcacagc tttggttact gaacttttgg attagtgcat ttatgaactt attggcttta    840
   aaaattttgc atgctgaata ccaatattat gtcccttctc agaattctat aactacagtg    900
   tcattattgt aataagactt ttgcatccat tgacaatggt atttgatact tttatagttt    960
   ctactattgt tagccaaagt tataaaacaa ataataaaat aacgttgaat caaaaaaaaa   1020
   aaaaaaaaaa gcggccgcgg atccccgggt aaaaggaatg tctcccttgc cagtactgct   1080
   agggtttttc tttcaaacta tggga                                          1105
<210> 3
   <211> 1145
   <212> DNA
   <213> fission yeast
<400> 3
   atttcagacg caattcacat ggcttttgac tgtattgcta ttcttgtcgg tttagttgct    60
   acgacgcttg ccaagatgcc tctaaattat gcttacccct ttggatttgc aaaaattgag   120
   gctctttcgg gtttcactaa tggtattttt ttagttttga tttcattttc tatcgtcggc   180
   gaggcattat ataggttatt tcatccgccc caaatgaata ccgaccaatt gttgttggtt   240
   agttttttgg gccttgttgt gaatttggta ggtatcctag cgttcaatca tgggcataat   300
   catgatcatg ggtctcatca ccatcattcc catagtaatc atagtatgtg tctgcctaac   360
   actacaaatg atataaatat ttttgaagag tttgaagaag aaaaagataa tgttgaagcc   420
   cagaaaatgg gctatacgaa tgacgatcac gtatcccaac atgaacatac ccatgagaat   480
   agtcaggaac atcaccatga gcataaccac aatcatgatc acatccataa atacaatgaa   540
   aaatgcgacc atgaaagcat aagtctccag aatttagaca atgatcatca ctgtcatcat   600
   caccatgaaa atcataatat gcatggcata tttctgcata ttatcgcaga tactatgggc   660
   tctgttggag ttattgtctc tactatatta atacagtggt tttcatggac cggttttgat   720
   ccttcggcat ctctaataat tgctgcatta atatttgttt ctgtacttcc attaattaaa   780
   gattcggcga agaatttgct ctctgtgact gatccagaat cggaatattt attgaagcag   840
   tgtttgtcga acatcagttt aagtcactcc gttgtcagtt tatccaaccc taagttctgg   900
   acaaacgaaa gaggtgaagt gtatggaata ctccatattc aggtgagcat agacggtgat   960
   ttaaacgtgg ttcgtaatga agtatttagg aagctctcaa tcgctgtacc aaatttaaaa  1020
   cacatttgta tacaatctga acggccaaac aattgctggt gtggaaaata gttcttacat  1080
   cagttgatat ccatacttat ttacgtgtaa ttttaattag atgaattaat attttcttta  1140
   ttagc                                                              1145
<210> 4
   <211> 906
   <212> DNA
   <213> fission yeast
<400> 4
   tttactttag tcgctttgtt aaattggcct cgaggtcgac gttaattaag cttttttttt    60
   aagagatata acatatgtca acgcgtcatt gattaactac ataacacgcc aattataaac   120
   ttctcccaaa agaacttaag aatttccatt ttcaatccag atgaatttat ttaagagacg   180
   aacagtagcg gcagcagcct tagcacgctt agcgagcaaa gcttgggtct taccacccat   240
   gatacccacc accccactta cgacgggctt tcgtcgtact tagcagagaa gttagcatca   300
   acggcggaga caatagaagc gagttcgttc ttgtcttcct cacggacctc agtgacagct   360
   aaaacagcag cagtcttttg gtgaatgaca gtaccaaggc gggccttgtt cttgacaatg   420
   gcataaggaa cacccatctt cttgcacaaa gcaggcaaga aaacgacgag ttcaatgggg   480
   tcgacatcgc tggcaatgag aaccaactta gccttcttgg cctcaatgag agctacaaca   540
   tggttcaaac catatttaac attgtaaggc ttcttagaga cgtcttgagc agacttgccg   600
   ttggcaacag cctcggcttc agcaaccaaa cgttgcttct tttcagcagc agtctcagga   660
   cggtacttgt taagcaactt gaagacctga gtagcagtgt ttttgtccaa agtcttctgg   720
   aactgagcaa tggcaggagg aaccttcaaa cgcaagttca aaatcttgcg acggcgttga   780
   aggcggatat actcaggcca cttaacaaaa cggctcaagt cacgcttagg ttggatgtct   840
   tgtcccccgg gtaaaaggaa tgtctccctt gccagtactg ctagggtttt tcgttcgaat   900
   aaggcc                                                              906

## Description

### TECHNICAL FIELD

The present invention is concerned with methods for enhancing gene suppression in cells and in particular it is concerned with improved methods for enhancing RNAi-mediated gene silencing by manipulation of factors associated with RNAi. The present invention is also concerned with methods for identifying factors which down-regulate as well as those which up-regulate RNAi. It is also concerned with genetic constructs useful for enhancing or modulating gene silencing and cells harbouring such constructs.

### BACKGROUND OF THE INVENTION

The use of double-stranded RNA (dsRNA) to specifically interfere with gene expression has received considerable attention because of its demonstrated potency in a range of organisms, including some which have so far been genetically intractable. Termed RNA interference (RNAi), it has been implicated in viral defense, control of transpositional elements, genetic imprinting and endogenous gene regulation. It has been hypothesised to be the central mechanism in post-transcriptional gene silencing (PTGS), co-suppression, quelling, and antisense RNA-mediated gene suppression. One model that has been proposed is that dsRNA is fragmented into 21-25 nt species by dsRNA-specific nucleases, amplified by RNA-dependent RNA polymerase, and then dissociated and free to attack homologous mRNA by RNA nuclease-mediated degradation. The application of this technique will greatly facilitate the dissection of gene function and the validation of genes involved in disease states.

Recently at least two different strategies have been undertaken to identify the cellular proteins composing a proposed multi-protein complex involved in the recognition of dsRNA and the activation of dsRNA-mediated gene interference. The first involves the use of classical chemical mutagenesis or insertional mutagenesis to isolate mutants completely defective for RNAi and cloning of the relevant genes using complementation. These studies have been carried out in genetically tractable organisms such as plants, worms and fungi. The genetic screens described involve the use of RNAi systems in which the degree of suppression is complete. The mutagenesis produces mutants in which the RNAi effect is completely reversed indicating the loss of a cellular factor (function) required for the RNAi effect. Thus these genetic screens would most likely miss factors that have subtle effects or rate limiting or rate determining roles in RNAi.

The second strategy for finding key players in RNAi has involved the use of cell free assays. These in vitro reconstitution assays, on the other hand, identify cellular factors that impact on RNAi outside of the cellular context and therefore the cellular role of these factors must always be tested.

However, the major disadvantages of these strategies are that genes will not be identified if they are essential to the organism, nor will they directly identify gene activities which will enhance RNAi when overexpressed.

Thus there is a need for models which can demonstrate a range of RNAi efficacies, with both increasing and decreasing quantitative activities being selectable. This would enable the identification of factors which can enhance or reduce the gene silencing effect.

It is therefore an object of the present invention to overcome or at least ameliorate one or more disadvantages of the prior art, or provide a useful alternative.

### SUMMARY OF THE INVENTION

Through the use of a fission yeast model for the study of dsRNA-mediated gene silencing and in the search for factors involved in this process, it was surprisingly found that the natural level of RNAi activity can be enhanced by manipulating factors associated with RNAi activity or efficacy. Thus, it has been found that by increasing the steady-state levels of a target nucleic acid sequence in the presence of the same pool of the corresponding antisense sequence, or a part thereof, the antisense-mediated suppression was not only maintained, but enhanced. This is indicative of an RNAi-like mechanism of gene suppression. It has also been found that overexpression of certain sequences, named herein *R*NAi enhancing sequences (*res*)*,* (also referred to herein as anti-sense enhancing sequences - *aes*)*,* also had the ability to enhance RNAi.

This ability of RNAi activity to be enhanced in *S. pombe* provides a model system which enables the analysis of RNAi processes and the identification and study of factors which either up-regulate or down-regulate its activity. The system has been further used to identify RNAi enhancing gene sequences which increase PTGS efficacy when their resulting protein activities are augmented in vivo. The model used to exemplify the present invention and the methods described are also applicable to treatment of disorders in which gene expression requires more efficient modulation or silencing.

According to one aspect there is provided a method for inhibiting ex vivo the expression of a target nucleic acid in a *Schizosaccharomyces pombe* cell in a cellular preparation, which method comprises the steps of
(i) increasing or decreasing expression of a nucleotide sequence represented by SEQ ID NO 1 in the cell, and
(ii) prior, concurrently with or subsequent to performing step (i), introducing into the cell a molecule which is, or gives rise to, an anti-sense nucleic acid directed toward at least a portion of the RNA transcript of the target nucleic acid.

According to further aspect there is provided a method of increasing ex-vivo cellular susceptibility to anti-sense-mediated inhibition of target nucleic acid expression, which method comprises increasing or decreasing expression of a nucleotide sequence represented by SEQ ID NO 1 in a *Schizosaccharomyces pombe* cell in a cellular preparation that expresses said target nucleic acid, and prior, concurrently or subsequently introducing into the cell a molecule which is, or gives rise to, an anti-sense nucleic acid directed toward at least a portion of the RNA transcript of the target nucleic acid.

The invention provides a pharmaceutical composition comprising:
(i) an expressible nucleotide sequence represented by SEQ ID NO 1;
(ii) a nucleic acid encoding a molecule which is, or gives rise to, an anti-sense nucleic acid directed toward at least a portion of the RNA transcript of a target nucleic acid; and
(iii) a pharmaceutically acceptable carrier.

Such a composition may be used for treating a subject suffering from a disorder whose alleviation is mediated by inhibiting the expression of a target nucleic acid, or for inhibiting in a subject the onset of such a disorder.

The invention also provides an isolated *Schizosaccharomyces pombe* cell having increased susceptibility to anti-sense-mediated inhibition of a target nucleic acid expression, which cell (i) expresses a target nucleic acid and (ii) comprises an elevated level of a nucleotide sequence represented by SEQ ID NO 1, and has a molecule introduced therein which is, or gives rise to, an anti-sense nucleic acid directed toward at least a portion of the RNA transcript of the target nucleic acid, permitting the nucleotide sequence represented by SEQ ID NO 1 to increase the degree to which the anti-sense nucleic acid inhibits expression of the target nucleic acid.

In a further aspect the invention provides an RNAi factor which is a *res* sequence represented by SEQ ID NO. 1 and a pharmaceutical composition comprising a *res* sequence represented by SEQ ID NO. 1 together with a pharmaceutically acceptable carrier.

In a preferred embodiment the target nucleic acid is an endogenous nucleic acid or a part thereof, but it may also be an exogenous sequence or part thereof

Preferably the expression of the sequence SEQ ID NO 1 is increased or decreased by introducing into the cell additional copies of, or agents which give rise to, the sequence. It will be understood therefore that up-regulating the expression of such a sequence will also achieve the same result and is contemplated herein as part of the invention.

Preferably the antisense nucleic acid corresponds to a part only of the target nucleic acid.

The term "inhibiting expression of a target nucleic acid" as used in the context of the present invention is intended to encompass, but not be limited to, reduction or elimination of gene expression, whether or not the target nucleic acid is a gene, or a part thereof, introduced into the cell or it is an endogenous gene.

The term "RNAi factor" is intended to include in its scope any naturally occurring, modified or synthetic molecule capable of enhancing RNAi activity. This definition includes in its scope the factors referred to herein as *R*NAi enhancing sequences (*res*)*.* The term *res* may be used herein interchangeably with the term *aes* (*a*nti-sense enhancing sequences).

It will be understood by those skilled in the art that "elevating RNAi factor level" can be achieved by transfection, upregulation or other means known in the art.

The term "anti-sense nucleic acid molecule" as used in the context of the present invention is intended to encompass RNA or DNA and contain region(s) related to a specific target RNA transcript or its gene. It is also intended to include molecules giving rise to antisense sequences, including inverted repeat, sense RNA, etc.

The term "conditions permitting the RNAi factor to increase the degree to which the anti-sense nucleic acid molecule inhibits expression of the gene" is intended to include the concept of inhibition of gene expression which is high enough to be effective, but not so high as to harm the cell. The effective concentration range can be determined using routine methodology known in the art.

Reference to a "subject" is intended to include human, animal (mouse), plant, or other life forms.

Reference to a "cell" is intended to encompass eukaryotic cells such as yeast, mammalian, plant, etc

The term "disorder" will be understood to inlcude viral infection (HIV, HPV, etc), cancer, autoimmune disease and other chronic and acute diseases.

The term "phenotype" as used in the context of the present invention is intended to include cell staining, morphology, growth, and similar characteristics.

### BRIEF DESCRIPTION OF THE FIGURES

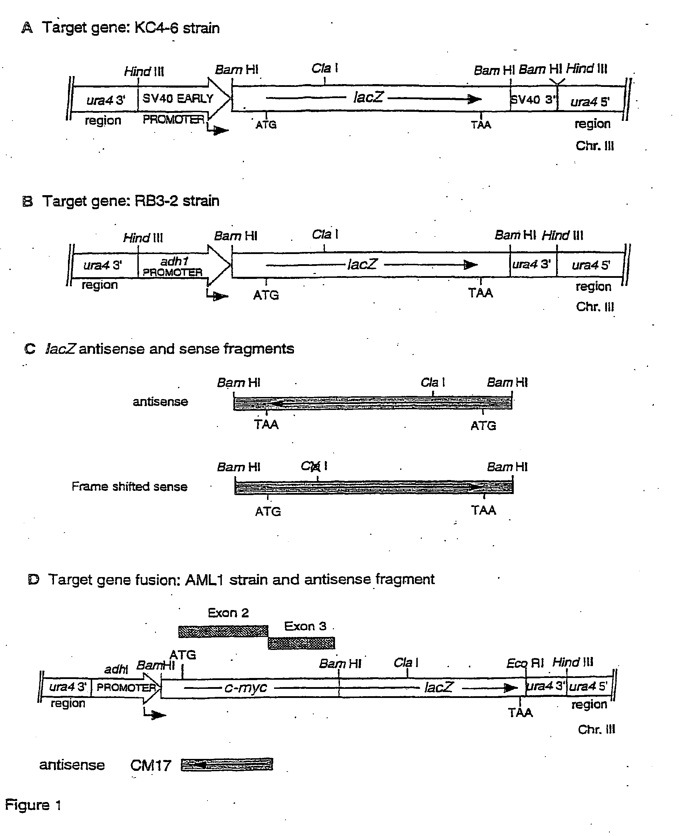

**Figure 1****.** Target genes and antisense fragments. **a**, The strain KC4-6 expresses low levels of *lacZ* RNA. The *lacZ* expression cassette is integrated at the *ura4* locus on chromosome III and is composed of the SV40 early promoter, the *E. coli lacZ* gene and the SV40 3' processing signal. The 5' and 3' flanking DNA sequences of the *ura4* locus are as indicated. **b**, The strain RB3-2, which expresses high levels of *lacz* RNA, contains an expression cassette containing the *S. pombe adh1* promoter, the *lacZ* coding region, and the *S. pombe ura4 3'* processing signal. **c**, The full-length 3.5 kb antisense *lacZ* fragment is shown. The crippled sense fragment was generated by end-filling the *Cla*I cut *lacZ* vector and religating on itself. **d**, The AML1 strain contains a *c-myc-lacZ* fusion cassette integrated at the *ura4* locus. Exons 2 and 3 of the human *c-myc* gene were employed as a target. The relative position of the *c-myc* antisense fragment is shown. Transcription initiation sites are indicated by the bent arrows. The straight arrows represent the normal direction of transcription for a particular DNA fragment.

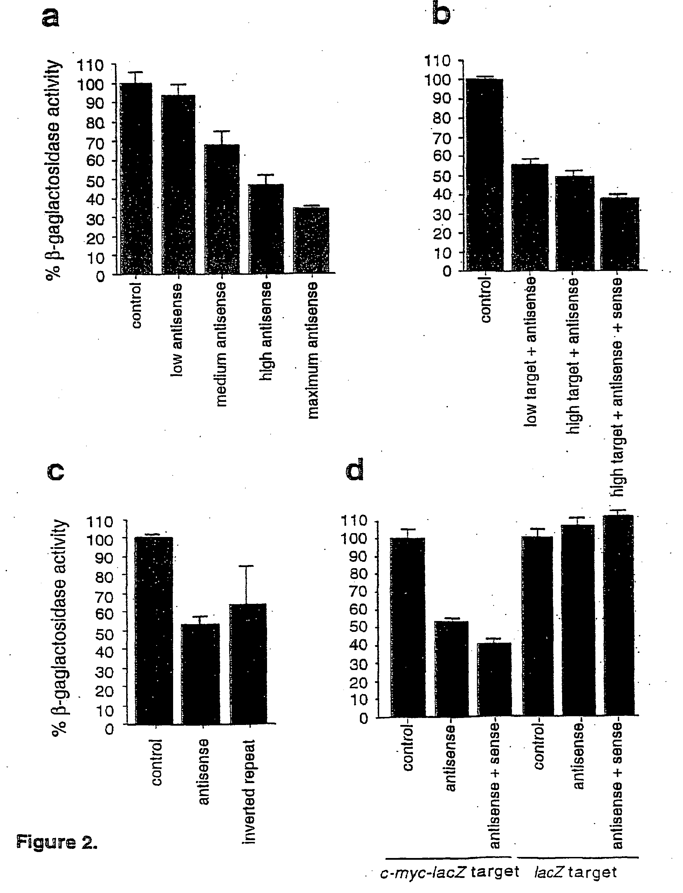

**Figure 2****.** dsRNA-mediated gene silencing in fission yeast. **a**, A fission yeast strain containing the integrated *lacZ* gene under control of the strong *adh1* promoter was transformed with *lacZ* antisense vectors. The antisense gene was under control of the weak *nmt41* promoter (low antisense), the strong *nmt1* promoter in single copy (15) (medium antisense), or the *nmt1* promoter in multi-copy (high antisense). For the strain expressing maximum antisense RNA two episomal *nmt1*-driven antisense vectors containing different selectable markers were co-transformed into the target strain. **b**, A fission yeast strain containing the integrated *lacZ* gene under control of the weak SV40 promoter (low target) or strong *adh1* promoter (high target) was transformed with the antisense *lacZ* vector or antisense *lacZ* and sense *lacZ* vectors. Strains were co-transformed with vectors containing appropriate selectable markers to complement auxotrophy. **c**, The *lacZ* inverted repeat vector was expressed in the high-expressing *lacZ* target strain. The strain expressing the antisense construct only is also indicated. **d**, A fission yeast strain containing an integrated *c-myc-lacZ* fusion gene was transformed with the sense construct, the antisense construct, or both. Antisense and sense constructs were also expressed in the high-expressing *lacZ* strain. All strains were transformed with appropriate control plasmids to complement auxotrophy.

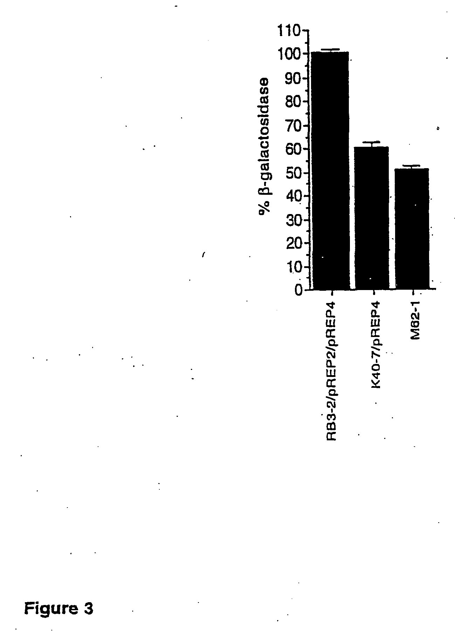

**Figure 3****.** Effect of co-expressing single copies of sense and antisense genes. Strains containing target *lacZ* alone (RB3-2), the target and a single copy of the antisense gene (K40-7), and the target and both the antisense and frameshifted *lacZ* genes (M62-1) were assayed for β-galactosidase activity. pREP2 and pREP4 are parental plasmids containing the *LEU2* and *ura4* selectable markers respectively. Strains were transformed with the appropriate control plasmids to complement auxotrophy. For each sample three independent colonies were assayed in triplicate.

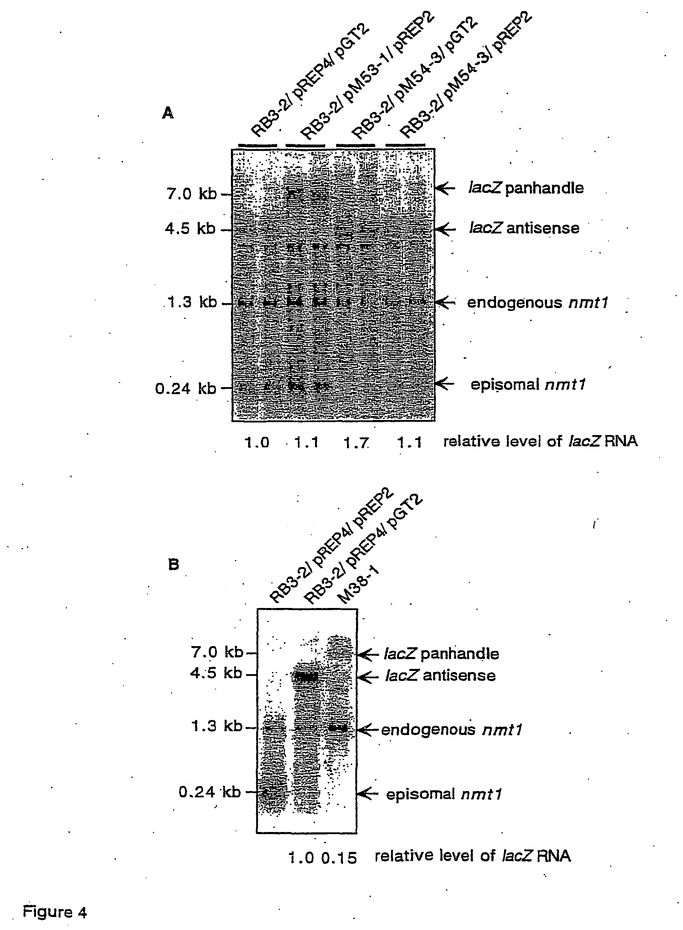

**Figure 4****.** RNA expression profiles in fission yeast strains. **a**, The target strain RB3-2 was transformed with the *lacZ* inverted repeat vector (pM53-1) or co-transformed with antisense (pGT2) and sense (pM54-3) vectors and grown to mid-log in the absence of thiamine. Total RNA from each strain was separated on a 1% denaturing agarose gel, transferred to a nylon membrane and probed with the radioactively labeled 2.2 kb *nmt1* fragment. The episomal *lac*z signal was normalized with the endogenous *nmt1* transcript and quantitated by phosphorimage analysis. The relative level of episomal *lacZ* expression is shown. **b**, The strain M38-1 which contains a single copy stably integrated *lacZ* inverted repeat gene was grown to mid-log in the absence of thiamine, its RNA isolated and analyzed by Northern hybridization. The relative steady-state level of the "panhandle" *lacZ RNA* is indicated in comparison to episomally expressed antisense *lacZ* RNA.

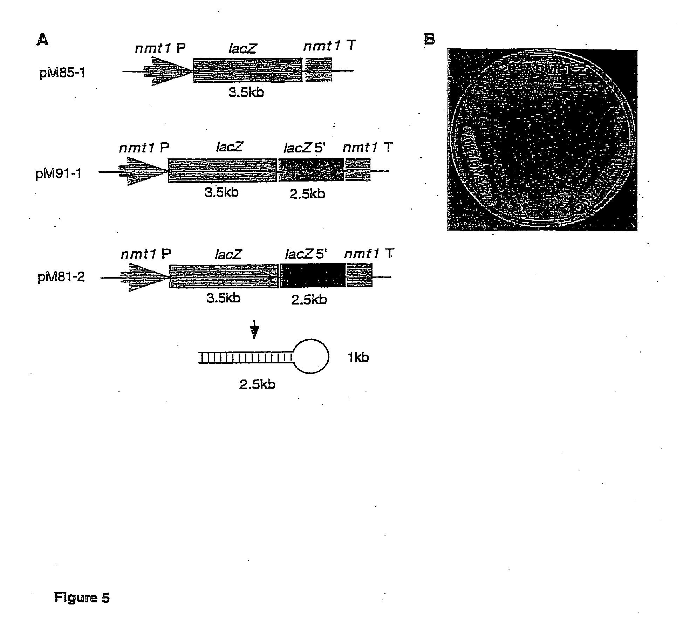

**Figure 5****.** In vivo dsRNA assay. **a**, The constructs employed for assaying the ability of the *lacZ* inverted repeat to form an intramolecular RNA duplex are shown. Each vector contains a functional *lacZ* fragment which gives the transformed strain a blue color phenotype when grown in the presence of X-GAL. The predicted secondary structure of the pM81-2 generated transcript is indicated. **b**, The strain 2037 was transformed with the vectors pM85-1, pM91-1 and pM81-2 in the absence of thiamine. Single colonies were streaked on minimal media plates and overlayed with 0.5% agarose medium containing 500 µg/ml X-gal and 0.01% SDS.

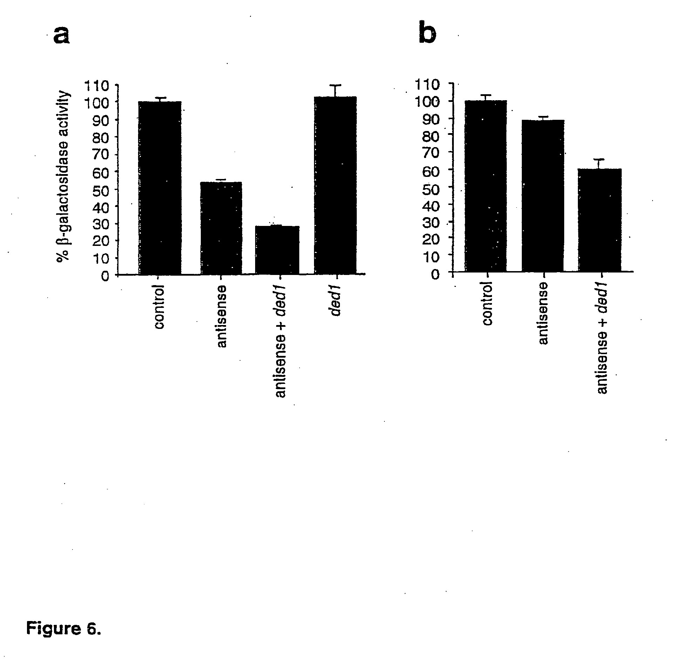

**Figure 6****.** The effect of *ded1* on dsRNA-mediated gene regulation. **a**, The *ded1* gene was co-expressed in a fission yeast strain containing the integrated *lacZ* gene. β-galactosidase activity of the strain transformed with and without the antisense *lacZ* vector is shown. **b**, The target strain was transformed with a vector expressing a short *lacZ* antisense gene or with both the short *lacZ* antisense vector and the *dedl*-expressing vector. Strains were transformed with appropriate control plasmids to complement auxotrophy.

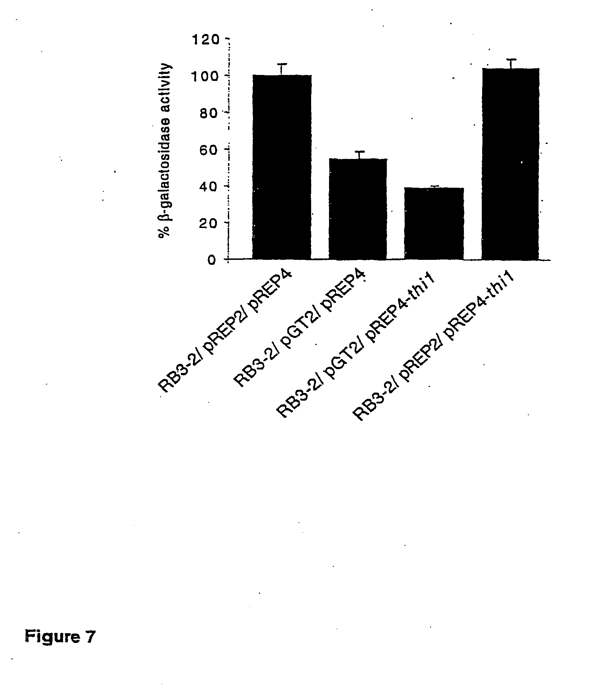

**Figure 7**. Over-expression *of thi1* in an antisense *lacZ* expressing strain.
β-galactosidase activity of the target strain RB3-2 transformed with and without the antisense *lacZ* vector is shown. Over-expression of the *thi1* gene in the antisense expressing strain is also indicated. Strains were transformed with appropriate control plasmids to complement auxotrophy.

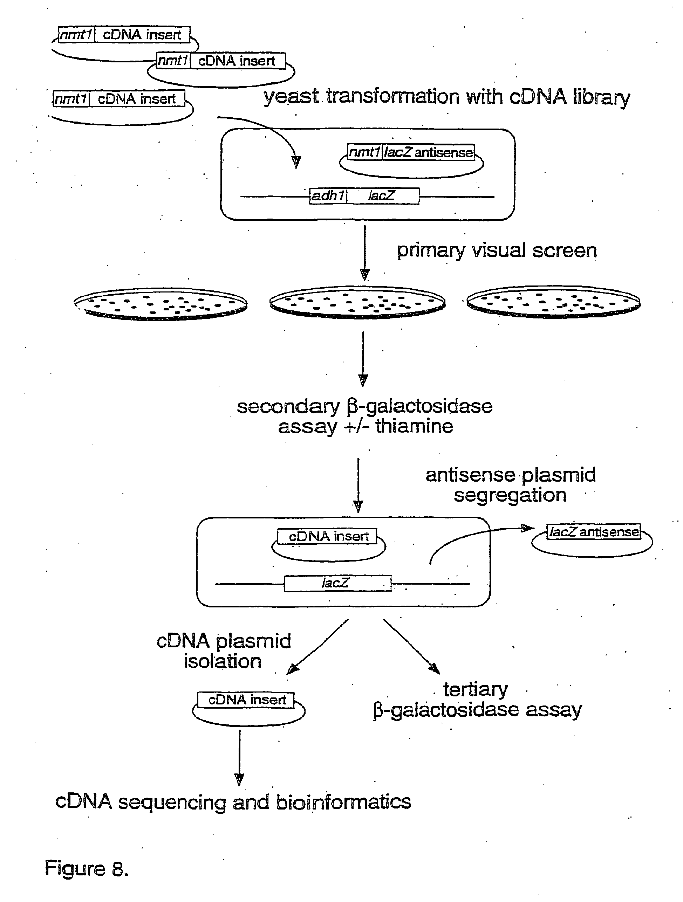

**Figure 8****.** Over-expression screening strategy for RNAi modulating factors. A target strain containing the integrated *lacZ* gene under control of the *adh1* promoter and the episomal vector containing the *nmt1*-driven *lacZ* antisense gene is transformed with an *S. pombe* cDNA library. Library fragments are driven by the *nmt1* promoter.
Transformants are individually screened for a change in the *lacZ-encoded* blue colony colour phenotype. These transformants are then quantitatively assayed for
β-galactosidase activity in the presence and absence of thiamine. The antisense vector is then segregated out of transformants showing a cDNA dependent modification of *lacZ* suppression. A tertiary β-galactosidase assay is performed to determine if the effect is dependent on the presence of dsRNA. cDNA vectors are recovered from strains of interest, sequenced, and subjected to BLASTN analysis.

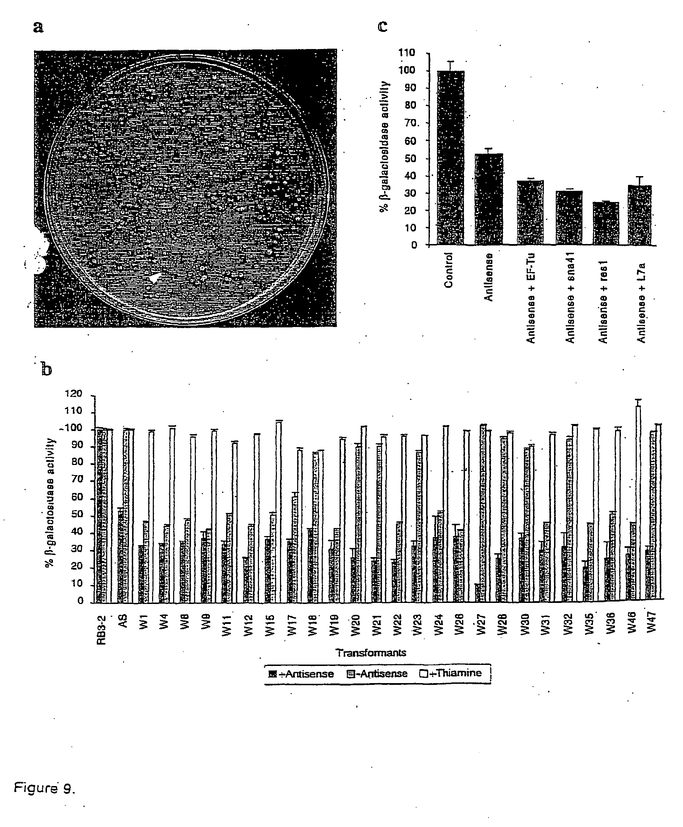

**Figure 9****.** Over-expression screen of a *S. pombe* cDNA library. **a**, Transformants were grown on minimal media plates and over-layed with X-GAL-containing medium. Those which showed a reduced blue colour-phenotype (black arrow) were analysed further.
Transformants demonstrating an enhanced blue-colour phenotype were also identified (white arrow). **b**, Transformants which showed a visual reduction in the blue phenotype were assayed for β-galactosidase activity in liquid culture in the absence of thiamine.
Thiamine was added to the medium to demonstrate that the enhanced *lacZ* suppression was dependent on RNA expression. Transformants were again assayed for
β-galactosidase activity following antisense vector segregation. **c**, Over-expression of unique *res* genes in dsRNA-expressing strains.

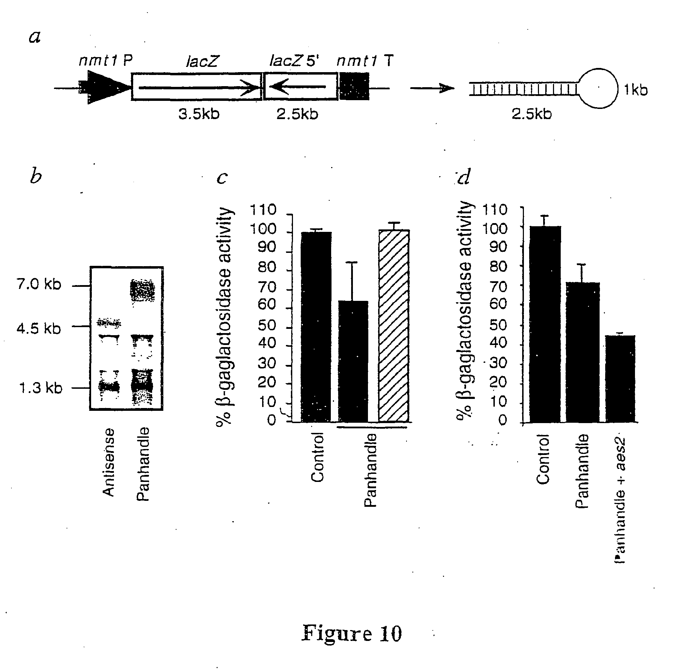

**Figure 10****.** lacZ panhandle-mediated gene silencing. (A) The lacZ panhandle construct contains the full-length crippled lacZ gene which is followed by the inverted 5'2.5 kb lacZ fragment. Intramolecular hybridization generates an RNA with 2.5 kb RNA duplex and a 1 kb loop. (B) The relative steady-state level of the episomally expressed panhandle lacZ RNA (7.0 kb) is shown in comparison to episomally expressed antisense lacZ RNA (4.5 kb). The lacZ signals were normalized to the endogenous nmt1 transcript (1.3 kb) and quantitated by phosphorimage analysis. (C) The target strain was transformed with the episomally expressed lacZ panhandle and analyzed for b-galactosidase activity. The appropriate plasmids were co-introduced to complement auxotrophy. At least three independent colonies were assayed in triplicate for each strain. Transformants were assayed in the presence of thiamine to abrogate expression of the panhandle RNA (hatched). (D) The target lacZ strain was transformed with the panhandle vector only or both the panhandle and aes2 vector.
**Figure 11****.** Co-expression of ded1 and lacZ antisense genes. b-galactosidase activity of the target strain co-transformed with the ded1 gene and the antisense lacZ vectors is shown. Ded1 was expressed from the ura4-based plasmid pREP4 while the antisense genes were expressed from the LEU2-based plasmid pREP2. Strains expressing antisense RNAs or ded1 RNA alone are also indicated. Strains were transformed with appropriate control plasmids to complement auxotrophy. Three independent colonies were assayed in triplicate for each strain.

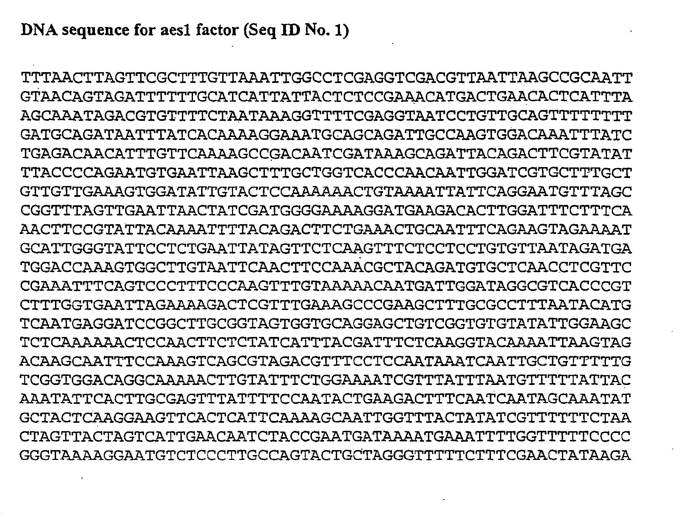

**Figure 12.** DNA sequence for *aes1* factor

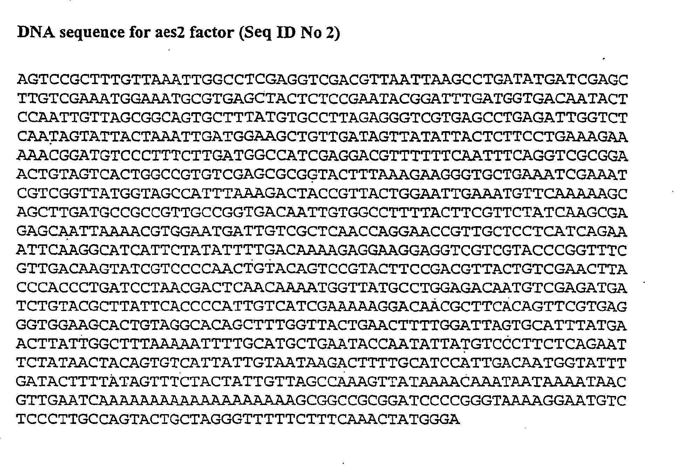

**Figure 13.** DNA sequence for *aes2* factor

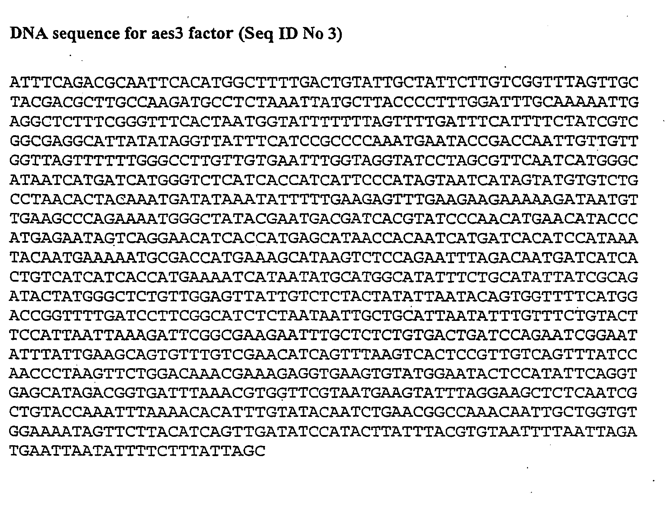

**Figure 14.** DNA sequence for *aes3* factor

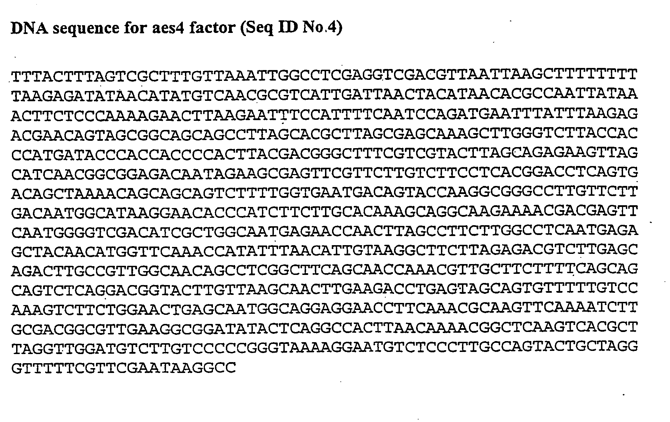

**Figure 15.** DNA sequence for *aes4* factor

### DESCRIPTION OF THE PREFERRED EMBODIMENTS

Using the *lacZ* fission yeast model to investigate features of antisense-RNA technology in vivo (1-4), it has been shown that the degree of target gene suppression is dependent on the level of antisense RNA (Fig. 2A)(1). During the course of developing this model it was suprisingly found that by increasing the steady-state levels of the *lacZ* target mRNA 20-fold in the presence of the same pool of antisense RNA that antisense-mediated suppression was not only maintained, but enhanced (Fig. 2B). To verify that this effect was independent of the target mRNA a *lacZ* gene containing a frameshift mutation (leading to an inability to translate the β-galactosidase enzyme) was co-expressed in antisense-expressing strains. Again, additional stimulation *of lacZ* inhibition was observed (Fig. 2B). These observations are consistent with the potential formation of additional dsRNA and consequent stimulation of gene silencing. They also indicate that the dsRNA-mediated gene silencing is dose-dependent in this system. To further show that dsRNA was the key component in target gene inhibition in fission yeast dsRNA was generated by expressing a *lacZ* inverted repeat with 2.5 kb of internal complementarity in the presence of *lacZ* target mRNA. Expression of this construct also reduced β-galactosidase activity in a *lacZ* expressing strain to a similar level to the full-length antisense RNA (Fig. 2C). Overall, these data suggested that increasing the potential formation of dsRNA, but not necessarily an antisense RNA:target mRNA hybrid, resulted in efficient interference of target gene expression in *S. pombe.*

To test the ability of dsRNA to specifically interfere with another target sequence in fission yeast, sense and antisense *c-myc* sequences were co-expressed in a strain containing an integrated *c-myc-lacZ* fusion cassette (3). A 792bp antisense *c-myc* fragment from exon 2 of the human *c-myc* gene was previously found to suppress β-galactosidase activity within the *c-myc-lacZ* fusion target strain by 47% (3). β-galactosidase assays demonstrated that co-expression of the antisense and sense *c-myc* constructs in the target strain enhanced *c-myc* suppression compared with the antisense *c-myc* vector alone (Fig. 2D). Transformation of the strain expressing only the *lacZ* target with the antisense and sense *c-myc* constructs resulted in no down-regulation of β-galactosidase activity indicating that the action of dsRNA is sequence-specific (Fig. 2D). All of the above observations, as well as the reduction in target mRNA levels in response to additional dsRNA (data not shown), are consistent with features associated with RNAi. This is the first example of dsRNA-mediated gene regulation in yeast and, unlike RNAi in all other organisms except zebrafish, appears to be dependent on the concentration of intracellular dsRNA. The variation in RNAi efficacy between organisms may be due to the absence or under-expression of some components of the RNAi pathway or the presence of RNAi inhibitors. We have now further developed this genetic model for identification of factors involved in RNAi.

Without wishing to be bound by any particular mechanism, it is probable that a multi-protein complex exists which mediates the post-transcriptional degradation of target mRNA in RNAi. Several genes which are involved in the RNAi phenomenon have been identified through genetic screens in *Neurospora* and nematodes. These genes include an RNA-dependent RNA polymerase (*qde-1, ego-1*) a RecQ DNA helicase *(qde-3),* an RNase D homologue (*mut-7*), and a putative translation initiation factor *(rde-1, qde-2).* The genes *mut-2, rde-2, rde-4,* and *rde-7* have also been identified as being involved in either the initiation or maintenance of RNAi. Additionally, *Drosophila* cell free assays have shown that RNAi is mediated by nuclease degradation of the targeted mRNA while a 21-25 nt RNA species appears to be integral to specific post-transcriptional genetic interference. RNAi has also been shown to be dependent on ATP which may be required for strand dissociation of dsRNA. However, a missing component of the proposed multi-protein complex in the RNAi pathway is the implied RNA helicase.

The dose-dependency of dsRNA-mediated gene silencing in fission yeast described above has allowed us to use an over-expression strategy to identify genes involved in RNAi. In comparison to mutagenesis strategies, over-expression can enable the identification of genes which are otherwise essential for cell viability. Also, cellular factors that quantitatively enhance or reduce RNAi activity can be determined. The first gene that was tested in mediating RNAi activity in the present model was the *mnt1* transcription factor *thi1.* This gene has been shown to specifically up regulate *nmt1* expression when overexpressed in fission yeast. As we have previously shown that antisense RNA-mediated gene suppression is dose dependent in *S. pombe* (1) it was hypothesized that over-expression of *thi1* would result in increased production of *nmt1-*driven antisense *lacZ* RNA and a consequent enhancement in target gene suppression. The *thi1* open reading frame was PCR amplified and subcloned into pREP4 as a *Bam*HI fragment. This vector was then transformed into RB3-2/pGT2 and β-galactosidase assays performed. As predicted, *lacZ* suppression was enhanced when compared to a strain expressing antisense RNA alone (Fig. 7). This result indicated that host-encoded factors are present which affect the robustness of dsRNA-mediated gene suppression in fission yeast and also validated the overexpression strategy.

The second gene investigated has been the *S. pombe* ATP-dependent RNA helicase gene, *ded1. Ded1* is an essential gene which has previously been characterized as a suppressor of sterility, a suppressor of checkpoint and stress response, and a general translation initiation factor. According to current models of dsRNA-mediated gene regulation an ATP-dependent RNA helicase may be required in conjunction with a dsRNA-dependent RNA polymerase for the formation of short single-stranded RNA fragments which specifically degrades target mRNA. It was therefore reasoned that over-expression of this gene in fission yeast could enhance the efficiency of dsRNA-mediated gene silencing by stimulating the unwinding of dsRNA. Co-expression of the *ded1* vector with the antisense *lacZ* vector significantly enhanced dsRNA-mediated *lacZ* inhibition by a further 50% compared to the antisense expressing strain (Fig. 6A). When *ded1* was over-expressed in the absence of the antisense *lacZ* vector, β-galactosidase activity was comparable to control strains indicating that its effect was dependent on the presence of dsRNA (Fig. 6A). The *ded1* vector was also co-expressed with a short antisense *lacZ* vector which has previously been shown to be less effective than the full-length antisense gene (16). Again, the over-expression of *ded1* stimulated dsRNA-mediated *lacZ* inhibition (Fig. 6B). It was thus shown that over-expression of an ATP-dependent RNA helicase greatly enhances RNAi in fission yeast.

By over-expressing a cDNA library in a dsRNA fission yeast model we have identified four novel genes with potential roles in the RNAi pathway. All of the known genes identified are essential and have natural associations with DNA, RNA or nucleic acid binding proteins, consistent with the expectation of RNAi cellular factors. In addition, two of the three known cDNAs encoded proteins involved in the process of translation. These findings along with our previous identification of an ATP-dependent RNA helicase (Raponi & Arndt, submitted) and reports of the involvement of a translation initiation factor in RNAi and the co-purification of ribonuclease activity with ribosomal fractions, suggests that this may be one site at which RNAi functions. Alternatively, the identification of such varied proteins as DNA helicases and translational components as part of the RNAi machinery implies that particular cellular proteins may be recruited into more than one multiprotein complex. Under the latter conditions, over-expression of these specific proteins may result in the generation of an RNAi complex that recognises dsRNA and mediates target gene suppression. Certain of these proteins may be rate-limiting or rate-determining in RNAi and only through supplementation of these factors are their roles in RNAi uncovered. It is proposed, without wishing to be bound by any particular mechanism, that in addition to the previously identified core proteins in the RNAi multiprotein complex(es), additional factors such as EF Tu, L7a, sna41 and res1 may be enlisted for a role in dsRNA-mediated gene suppression.

The over-expression strategy described overcomes some limitations associated with mutagenesis by identifying essential genes with a role in RNAi. In addition, this strategy complements these other systems by allowing the isolation of cellular factors that modify the efficacy of RNAi in vivo. The roles of these modulators of RNAi may be varied and include recognition and amplification of the dsRNA, delivery of the small 21-25 nt dsRNAs to the target mRNA, association between the antisense and target mRNA strands, and RNAi complex formation. Alternatively, these modulators may control the rate of RNAi or the formation of different complexes within cell types or for different forms of post-transcriptional gene silencing. The fact that over-expression of specific RNAi modulators enhanced dsRNA-mediated gene regulation in fission yeast indicates that a similar approach could be used to identify RNAi modulators in other organisms. In addition, the co-expression of these factors with different forms of post-transcriptional gene silencing including co-suppression, quelling, and antisense RNA could be one way of enhancing the efficacy of these methods. This may be especially important for application of RNAi to mammalian cells and tissues or to genes which have been somewhat recalcitrant to this form of regulation.

The present invention demonstrates for the first time the intrinsic involvement of an ATP-dependent RNA helicase as a key component in RNAi. Further, it can be rate limiting, as the over-expression leads to increased RNAi activity in this system. This ability of the *ded1-*encoded RNA helicase is consistent with its activities as a member of the DEAD box family of helicases, with their three core domains of ATPase, RNA helicase, and RNA binding activities. These could allow the enzyme to enhance gene suppression as follows: (i) in a dissociative mechanism it could mediate either the unwinding of dsRNA to generate a cRNA in conjunction with an RNA-dependent RNA polymerase or strand separation of fragmented dsRNA to enhance binding to homologous transcripts, and/or (ii) in an associative mechanism it could catalyse the ATP-dependent exchange of the sense strand of the short dsRNA with the target mRNA. The presence of *ded1* homologues in other organisms displaying RNAi further supports the general involvement of this component in the RNAi machinery.

The present invention also provides for the first time a novel and quantitative genetic system based on *S.pombe,* for rapidly identifying essential cellular factors involved in RNAi. The use of this model has enabled verification of RNA helicase activity as a critical contributor to efficient RNAi activity and isolated novel RNAi factors including EF Tu, L7a, *sna41,* and an unidentified gene *res1.* The present invention also demonstrates that gene silencing may be enhanced by concomitant expression of such RNAi factors.

The invention will now be described more particularly with reference to nonlimiting examples of certain preferred embodiments of the invention.

### EXAMPLE 1: Materials and methods used to exemplify the invention

### S. pombe media and manipulations.

All yeast strains were maintained on standard YES or EMM media (6). Repression of *nmt1* transcription was achieved by the addition of thiamine to EMM media at a final concentration of 4 µM (7). Yeast cells were transformed with plasmid DNA by electroporation (8) and stable integrants were identified as previously described (6). A glass bead procedure (9) was used to isolate genomic DNA which was used for Southern analysis and PCR diagnosis. Total RNA was extracted as previously described (10).

### S. pombe strain and plasmid construction.

Construction of the low expressing *lacZ* strain, KC4-6 (*h⁻, ura4::SV40-lacZ, leu1-32*), has been previously described (2). The strain RB3-2 (*h-, ura4::adh1-lacZ, leul-32)* which expresses higher levels of β-galactosidase has also been previously described (1). The target strain which contains the *c-myc:lacZ* fusion has previously been described (3).

The construction of the long *lacZ* antisense containing episomal plasmid, pGT2, and corresponding control plasmids have been described (2). Plasmid pREP4-As was generated by subcloning the *lacZ Bam*HI fragment contained in pGT2 into the plasmid pREP4 (11). pREP4 is identical to pREP1 except that the *S. cerevisiae LEU2* gene has been replaced with the *S. pombe ura4* selectable marker. To decrease the steady-state level of episomally expressed antisense *lacZ,* plasmids pREP42-As and pREP82-As were constructed by subcloning the *lacZ Bam*HI fragment from pGT2 into pREP42 and pREP82, respectively. These plasmids are derivatives of pREP4 with mutations in the TATA box of the *nmt1* promoter (12). The crippled *lacZ* vector, pGT62, was generated by end-filling the *Cla*I site of pGT2 and re-ligating (2). This frameshifted fragment was then subcloned into the *Bam*HI site of pREP4 to generate the plasmid pM54-3.

The *lacZ* panhandle integration vector, pM30-8, was generated by first introducing a *Not*I site into the *Xma*I site of pRIP1/s (11) using the self-complementary linker 5' CCG GGC GGC CGC 3' to generate pL121-14. The 2.5 kb sequence of the 5' end of the frameshifted *lacZ* gene (2) was then PCR-amplified to give it *NotI* ends using the forward primer 5' ATGCGGCCGCAATTCCCGGGGATCGAAAGA 3' and reverse primer 5' ATGCGGCCGCAATGGGGTCGCTTCACTTA 3'. This product was cloned using a TA cloning kit (TOPO: Invitrogen, San Diego, CA, USA) and then subcloned into the *Not*I site of pL121-14 in the antisense orientation. The integrating vector was introduced into target strains in single copy by using the *sup3-5*/*ade6-704* complementation system (13). The full-length frameshifted *lacZ* fragment was then introduced into the *BamH*I site of this vector in the sense orientation upstream of the 2.5 kb antisense fragment to generate pM30-8. The episomal version of this vector (pM53-1) was made by removing the *Pst1 sup3-5* fragment and introducing the autonomous replicating sequence as an *Eco*RI fragment (11). For testing the ability of the panhandle construct to form dsRNA in vivo, the frame shifted *lacZ* fragment was replaced with the functional *lacZ* fragment in the vectors pM54-3 and pM53-1, to generate the vectors pM85-1 and pM81-2, respectively. The vector pM91-1, which is unable to form a *lacZ* panhandle transcript, was generated by removing the 2.5 kb *Not*I *lacZ* fragment from pM81-2 and reintroducing it in the sense orientation.

Generation of the *c-myc* antisense construct, pCM-17, has been described elsewhere (3). The 792 bp *Bgl*II *c-myc* fragment from pCM-17 was subcloned into the *Bam*HI site of pREP4 in the sense orientation to generate pN12-1.

The *ded1,* and *thi1,* open reading frames were amplified from fission yeast genomic DNA (strain 1913). *ded1* was amplified to give it *Bam*HI ends using the forward primer 5' ATGGGATCCCAACCAAACACTTCAACTCAG 3' and the reverse primer 5' ATGGGATCCTCAGAAGCCTGTGCATAACAC 3'. *thi1* was amplified to give it *Bgl*II ends using the forward primer 5' ATGAGATCTGTGGTTGGTATTCTAGAGAGA 3' and the reverse primer 5' ATGAGATCTAACAAAGACCTGCAAAAAACC 3'. PCR products were purified (Qiagen PCR purification kit), digested with either *Bam*HI or *Bgl*II*,* gel-purified (Qiagen), and subcloned into the *Bam*HI site of pREP4 in the sense orientation.

### Southern and Northern analysis.

Nucleic acid electrophoresis and membrane transfer was performed as described (14). Southern and Northern blots were hybridized using ExpressHyb solution according to the manufacturer's instructions (Clontech Laboratories). DNA probes were ³²P-labelled using the Megaprime labelling kit (Amersham). Probes included a 960 bp *Bam*HI/*Cla*I *lacZ* fragment from pI2-1 (1), a 570 bp *Hind*III/*Eco*RI *ura4-3'*fragment from pGT113 (15), and a 2.2 kb *Pst*I/*Sac*I *nmt1* fragment from pRIP1/s. Radioactive signals were detected by autoradiography and quantitated by phosphorimage analysis (ImageQuant; Molecular Dynamics).

### Plasmid distribution analysis.

Plasmid co-transformants of strain RB3-2 were grown under selective conditions to a cell density of 1-2x10⁷ cells/ml. A serial dilution of each culture was performed and cells plated for single colonies in triplicate onto each of YES, EMM, EMM+leucine, and EMM+uracil agar media. The number of colonies grown on YES was taken as the total number of viable cells, while colonies growing on EMM represented the cells in the sampled population that contained both the *ura4*-containing (pREP4-based) and the *LEU2*-containing (pREP1-based) plasmids. Cells containing either the *ura4*-containing plasmid or the *LEU2*-containing plasmid were identified from the EMM+leucine and EMM+uracil plates respectively. The ratio of the number of colonies grown on selective media to the total number of viable colonies was used as the quantitative measure of the proportion of cells in the population, grown under selection, which contained plasmids.

### β-galactosidase assays.

The expression of the *lacZ* gene-encoded product, β-galactosidase, was quantitated using a cell permeabilization protocol as previously described (Raponi et al., 2000). A semi-quantitative overlay assay was also employed for rapid screening of yeast transformants (3).

### Isolation of S. pombe cDNA clones that alter antisense RNA efficacy.

The *S. pombe* cDNA library was originally constructed in pREP3Xho by Bruce Edgar and Chris Norbury (5). The vector pREP3Xho is derived from pREP3 which contains the *LEU2* marker and inserts are under control of the *nmt1* promoter (11). A total of 5 µg of library DNA was transformed into the strain RB3-2 containing the episomal antisense *lacZ* vector pREP4-lacZAS and grown in EMM liquid media to the mid-logarithmic phase. Transformants were then plated on EMM solid media and grown at 30°C for 3 days. Colonies were over-layed with medium containing 0.5 M sodium phosphate, 0.5% agarose, 2% dimethylformamide, 0.01 % SDS, and 500 µg/ml X-GAL (Progen, Australia). Plates were then incubated at 37°C for 3 hrs, colonies of interest recovered and assayed for β-galactosidase activity.

### Plasmid segregation.

Strains were plated on EMM containing limiting uracil and 1 mg/ml 5-fluoroorotic acid (6). Strains were then replica plated on both selective and non-selective media. Those colonies that did not grow on selective media were identified as having lost the *ura4-*containing antisense plasmid. To determine the mitotic stability of pREP-based plasmids we used the plasmid segreagtion method previously described (16). This method indicated that up to 30% of the cells grown in selective media did not contain the resident plasmid.

### EXAMPLE 2: Effect of steady-state level of target mRNA and antisense RNA on antisense efficacy.

To determine the role of target mRNA steady-state levels in antisense-mediated gene suppression, we investigated the ability of a long *lacZ* antisense RNA (2) to regulate *lacZ* target genes under control of both weak and strong constitutive promoters. The low-level expressing strain, KC4-6, contained the *lacZ* gene driven by the SV40 early promoter integrated at the *ura4* locus in chromosome III (Fig. 1A; (2)). The high-level *lacZ* expressing strain, RB3-2, was constructed by placing the *lacZ* gene under control of the strong fission yeast *adh1* promoter and the 3' processing signal from the *ura4* gene and integrating this expression cassette at the *ura4 locus* (Fig. 1B; (1)). Characterization of both strains indicated that RB3-2 expressed 20-fold more *lacZ* mRNA than KC4-6 while an approximate 40-fold increase in β-galactosidase was also detected (data not shown). Both strains were transformed with the *lacZ* antisense gene-containing plasmid (pGT2) and its corresponding sense control plasmid (pGT62). β-galactosidase assays indicated that the antisense RNA suppressed β-galactosidase activity by 45% in KC4-6 while the same RNA reduced β-galactosidase activity by 52% in RB3-2 (Fig. 2B). No reduction in β-galactosidase activity was seen in either strain when transformed with the control plasmid pGT62 which expresses a crippled version of *lacZ.* These results indicated that despite a 20-fold increase in the steady state level of the *lacZ* target mRNA in RB3-2 the efficacy of antisense-mediated down regulation was not only maintained, but enhanced. The increase in gene suppression demonstrated with strain RB3-2 was also seen when antisense molecules targeted to subregions of the *lacZ* target gene were used, but was not as pronounced. These data suggested an increase in target mRNA can result in enhanced target gene suppression.

It has previously been demonstrated that a stably integrated antisense *lacZ* gene acts in a dose dependent fashion with the steady-state levels of antisense RNA being dependent on genomic position effects and transgene copy number (1). Here the role of the steady-state level of episomally expressed antisense RNA in both the low *lacZ* expressing target strain, KC4-6, and the high *lacZ* expressing target strain, RB3-2, was investigated. The expression of *lacZ* antisense RNA was increased by co-transforming KC4-6 and RB3-2 with plasmids pGT2 and pREP4-As, each of which contains the *lacZ* antisense gene under control of the *nmt1* promoter, but different selectable markers. To decrease the steady-state level of antisense RNA, the plasmids pREP42-As and pREP82-AS were employed. The *nmt1* promoter in these vectors contain deletions in the TATA box sequence which affect the level of transcription, but have no impact on the site of transcription initiation or thiamine repressibility (12). Each of the different antisense gene-containing plasmids was co-transformed with a control plasmid to complement auxotrophy where appropriate. This resulted in a set of co-transformants of both RB3-2 and KC4-6 each containing the same *lacZ* antisense gene, but with different promoter capacities. Each co-transformant was analyzed for antisense RNA steady-state levels and β-galactosidase activity. Table 1 indicates that with both strains the degree of target suppression is enhanced with the increase of antisense *lacZ* RNA expression.

**Table 1. Effects of varying the level of antisense RNA in strains expressing both low and high level target.**

| Strain | Plasmids | Descriptions | β-gal. activity (units) | % suppression**^{a}** | Relative antisense RNA level**^{b}** |
|---|---|---|---|---|---|
| KC4-6 | pREP2, pREP4 | Vector | 1.07 | 0 | 0 |
| | pREP2, pREP82-AS | Low As | 1.10 | 0 | 0.04 |
| | pREP2, pREP42-AS | Medium As | 1.07 | 0.1 | 0.14 |
| | pREP2, pREP4-AS | Full As*-ura4* | 0.75 | 30 | 1.0 |
| | pGT2, pREP4 | Full As*-LEU2* | 0.70 | 34.6 | 2.2 |
| | pGT2, pREP4-AS | Double-As | 0.54 | 49.2 | 5.6 |
| RB3-2 | pREP2, pREP4 | Vector | 48.5 | 0 | 0 |
| | pREP2, pREP82-AS | Low As | 44.0 | 9.4 | 0.05 |
| | pREP2, pREP42-AS | Medium As | 43.9 | 9.6 | 0.18 |
| | pREP2, pREP4-AS | Full As*-ura4* | 27.2 | 43.9 | 1.0 |
| | pGT2, pREP4 | Full As*-LEU2* | 24.9 | 48.7 | 1.9 |
| | pGT2, pREP4-AS | Double-As | 16.6 | 65.8 | 5.4 |

| | | | | | |
|---|---|---|---|---|---|
| **a**. The % suppression of β-galactosidase activity was determined by expressing each β-galactosidase activity as a percentage of the activity found in the pREP2,pREP4 co-transformant. **b**. Within each strain the steady-state level of long *lacZ* antisense RNA was normalized to *nmt1* mRNA and then expressed relative to the level observed in the pREP2, pREP4-As co-transformant. | | | | | |

These data agree with the previous report that antisense RNA-mediated gene inhibition is dose dependent in *S. pombe* (1). However, for each co-transformant the degree of β-galactosidase suppression was greater by 10-15% in the high *lacZ* expressing strain, RB3-2, than the low *lacZ* expressing strain, KC4-6, again demonstrating that low levels of target mRNA can limit antisense efficacy.

### EXAMPLE 3: Effect of increasing sense RNA on antisense RNA-mediated target gene suppression.

To determine whether the increase in gene suppression was due to formation of an antisense RNA:target mRNA hybrid or an antisense RNA:sense RNA hybrid, a version *of lacZ* which is unable to be translated into functional β-galactosidase was co-expressed in strains expressing the *lacZ* antisense gene. If antisense RNA was required to hybridize to target mRNA for inhibition of the gene expression pathway, then over-expression of the sense RNA would compete with the target for the available antisense molecules and a decrease in *lacZ* gene suppression would result. Initially, both antisense and sense *lacZ* vectors were integrated in single copy into separate target strains and then crossed with each other. In strains containing the antisense gene alone, β-galactosidase activity was reduced by approximately 40% (Fig. 3; (1)) while there was no reduction in the strain expressing sense *lacZ* alone (data not shown). However, in the strain expressing both complementary transcripts (M62-1), β-galactosidase activity was reduced by 50% (Fig. 3). These results suggested that increasing the intracellular concentration of sense RNA in the presence of antisense RNA, and therefore potentially generating more dsRNA, stimulates target gene suppression.

To further increase the potential formation of intracellular dsRNA an episomal sense *lacZ* plasmid (pM54-3; Fig. 2B) was co-transformed with the episomal antisense *lacZ* plasmid, pGT2, into the target strain RB3-2. Both of these sequences were expressed by the strong conditional *nmt1* promoter and RNA analysis showed that each transcript was being expressed at high levels when grown in the absence of thiamine (Fig. 4A). When both RNAs were co-expressed in RB3-2, the target *lacZ* was suppressed by 65% compared to 50% in the strain expressing the antisense plasmid alone (Fig. 2B). Northern analysis demonstrated that the total relative level of episomally expressed *nmt1*-driven transgenes was approximately 60% higher than when either vector was expressed alone (Fig. 4A). Although expression of these complementary RNA transcripts is at a high level compared with single copy integrants only an additional 15% suppression was observed.

Once explanation for the absence of higher levels of target gene suppression is plasmid segregation. *S. pombe* undergoes asymmetric segregation, with the result that mitosis produces a daughter cell which lacks the segregating plasmid (17). Therefore, when the 65% suppression level seen in the total population is corrected for only those cells containing the *ura4*-based and *LEU2*-based plasmids the level of suppression approaches approximately 100% (data not shown). Overall these data suggest that increasing the potential formation of dsRNA, but not necessarily an antisense RNA:target mRNA hybrid, is required for efficient interference of target gene expression in *S. pombe.* However, unlike the phenomenon of RNAi seen in plants and nematodes, the dsRNA-mediated gene suppression demonstrated in fission yeast seems to be dependent on the concentration of intracellular dsRNA or a threshold level of dsRNA is required to invoke potent gene silencing.

### EXAMPLE 4: Effect of a lacZ inverted repeat on lacZ gene expression.

To further investigate the ability of dsRNA to inhibit gene expression in fission yeast we generated a vector containing the full-length 3.5 kb frameshifted *lacZ* sequence with a 2.5 kb inverted repeat. This construct generates a transcript of approximately 7 kb in length with 2.5 kb of self-complementarity which, predictably, will form a strong intramolecular RNA duplex. This gene was initially integrated into a fission yeast strain in single copy and then crossed with the strain RB3-2. The resulting strain which contained the single copy inverted repeat gene and target *lacZ* showed no reduction in β-galactosidase activity when transcription of the inverted repeat was activated. Southern analysis confirmed that the cassette was intact (data not shown) while RNA analysis indicated that the 7 kb transcript was being generated but approximately 10-fold less than episomally expressed antisense *lacZ* (Fig. 4A&B). To increase the steady-state level of the inverted repeat RNA we generated the episomal plasmid pM53-1 containing this cassette. RNA analysis demonstrated that this vector is expressed at a similar level to episomally expressed antisense *lacZ* (Fig. 4A). When expressed in RB3-2 pM53-1 inhibited β-galactosidase activity by 40% (Fig. 2C), while addition of thiamine to the culture medium returned β-galactosidase activity to control leve.

To confirm that gene inhibition was due to this construct forming an RNA duplex, an in vivo assay for dsRNA was developed. To this end, a series of vectors were generated which contained functional *lacZ* sequences including *lacZ* alone (pM85-1), a *lacZ* inverted repeat (pM81-2), and the *lacZ* repeat with both sequences in the sense orientation (pM91-1) (Fig. 5A). These vectors were then introduced into a strain lacking the integrated target sequence (NCYC2037; *h⁺, ura4-D18)* and resulting transformants were overlayed with X-GAL-containing agarose. Both strains containing control vectors generated high levels of β-galactosidase while the strain expressing the *lacZ* inverted repeat did not (Fig. 5B). In each case Northern analysis demonstrated that strains were expressing the transgene. These results indicate that the inverted repeat RNA could not be efficiently translated to produce β-galactosidase, most probably due to the formation of a strong intramolecular hairpin structure. Taken together, these results show that a construct which has the ability to form dsRNA by intramolecular hybridization will interfere with target gene expression when expressed at high levels, again indicating that dsRNA mediates target gene suppression in a dose-dependent manner in fission yeast.

### EXAMPLE 5: Gene-specificity of dsRNA-meditated gene silencing in fission yeast.

To test the ability of dsRNA to specifically interfere with other target sequences in fission yeast, sense and antisense *c-myc* sequences were co-expressed in a strain containing an integrated *c-myc-lacZ* fusion cassette (Fig. 1D; (3)). A 792bp antisense c-*myc* fragment from exon 2 of the human *c-myc* gene (named CM-17) was previously found to suppress β-galactosidase activity within a *c-myc-lacZ* fusion target strain (AML1) by 47% (3). CM-17 was subsequently subcloned into pREP4 in the sense orientation to generate pN12-1. The antisense *c-myc* vector (pCM-17) and the sense *c-myc* vector were transformed into AML1 both independently and together. β-galactosidase assays demonstrated that co-expression of the antisense and sense constructs enhanced *c-myc* suppression by an additional 13% compared with the antisense *c-myc* vector alone (Fig. 2D). Transformation of RB3-2 with the antisense and sense *c-myc* constructs resulted in no down-regulation of β-galactosidase activity indicating that the action of dsRNA is gene-specific (Fig. 2D). These results demonstrate that dsRNA can specifically interfere with multiple targets in fission yeast including one of human origin.

### EXAMPLE 6: Over-expression of a translation factor can enhance RNAi in fission yeast.

The dose-dependency of dsRNA-mediated gene silencing in fission yeast described above has allowed us to use an over-expression strategy to identify genes involved in RNAi. In comparison to mutagenesis strategies, over-expression can enable the identification of genes which are otherwise essential for cell viability. Also, cellular factors that quantitatively enhance or reduce RNAi activity can be determined. The first gene that we have tested in mediating RNAi activity in the present model has been the *S. pombe* ATP-dependent RNA helicase gene, *ded1. Ded1* is an essential gene which has previously been characterized as a suppressor of sterility, a suppressor of checkpoint and stress response, and a general translation initiation factor. According to current models of dsRNA-mediated gene regulation an ATP-dependent RNA helicase may be required in conjunction with a dsRNA-dependent RNA polymerase for the formation of short single-stranded RNA fragments which specifically degrades target mRNA. We therefore reasoned that over-expression of this gene in fission yeast could enhance the efficiency of dsRNA-mediated gene silencing by stimulating the unwinding of dsRNA. Co-expression of the *ded1* vector with the antisense *lacZ* vector significantly enhanced dsRNA-mediated *lacZ* inhibition by a further 50% compared to the antisense expressing strain (Fig. 6A). When *ded1* was over-expressed in the absence of the antisense *lacZ* vector, β-galactosidase activity was comparable to control strains indicating that its effect was dependent on the presence of dsRNA (Fig. 6A). The *ded1* vector was also co-expressed with a short antisense *lacZ* vector which has previously been shown to be less effective than the full-length antisense gene (2). Again, the over-expression *of ded1* stimulated dsRNA-mediated *lacZ* inhibition (Fig. 6B). We have thus shown that over-expression of an ATP-dependent RNA helicase greatly enhances RNAi in fission yeast. Although an ATP-dependent RNA helicase has recently been suggested to be a key component in RNAi, this is the first demonstration of its intrinsic involvement. Further, it can clearly be rate limiting, as the over-expression leads to increased RNAi activity in this system. This ability of the *ded1*-encoded RNA helicase is consistent with its activities as a member of the DEAD box family of helicases, with their three core domains of ATPase, RNA helicase, and RNA binding activities. These could allow the enzyme to enhance gene suppression as follows: (i) in a dissociative mechanism it could mediate either the unwinding of dsRNA to generate a cRNA in conjunction with an RNA-dependent RNA polymerase or strand separation of fragmented dsRNA to enhance binding to homologous transcripts, and/or (ii) in an associative mechanism it could catalyse the ATP-dependent exchange of the sense strand of the short dsRNA with the target mRNA. The presence of *ded1* homologues in other organisms displaying RNAi further supports the general involvement of this component in the RNAi machinery.

### EXAMPLE 7: Over-expression of a transcription factor can enhance RNAi in fission yeast.

The second gene investigated was the *nmt1* transcription factor *thi1.* This gene has been shown to specifically up regulate *nmt1* expression when overexpressed in fission yeast. As we have previously shown that antisense RNA-mediated gene suppression is dose-dependent in *S. pombe* (1) it was hypothesized that over-expression *of thi1* would result in increased production of *nmt1-driven* antisense *lacZ* RNA and a consequent enhancement in target gene suppression. The *thi1* open reading frame was PCR-amplified and subcloned into pREP4 as a *Bam*HI fragment. This vector was then transformed into RB3-2/pGT2 and β**-**galactosidase assays performed. As predicted, *lacZ* suppression was enhanced when compared to a strain expressing antisense RNA alone (Fig. 7). This result indicated that host-encoded factors are present which modulate the efficacy of dsRNA-mediated gene suppression in fission yeast and also validated the over-expression strategy.

### EXAMPLE 8: Screening for novel RNAi modulating factors.

Over-expression of a fission yeast cDNA library (5) in an antisense *lacZ* expressing strain, revealed a series of transformants in which β-galactosidase activity was significantly reduced from that demonstrated in antisense RNA-expressing strains alone. The screening strategy is shown in figure 8. Transformants were grown on selective plates and overlayed with X-GAL-containing media to generate a blue phenotype which could be analysed visually in a semi-quantitative manner (Fig. 9A)(3). From 12,000 transformants screened, 48 were initially identified as having a reduced blue phenotype compared with background transformants. Of these, 25 demonstrated a reproducible enhancement in antisense RNA-mediated *lacZ* suppression when quantified by a liquid β-galactosidase assay (Fig. 9B). To demonstrate that the observed effects were due to transcription of the cDNA insert, transformants were grown in the presence of thiamine. This repression of the *nmt1* promoter resulted in a return to control levels of β-galactosidase activity in all transformants (Fig. 9B). This indicated that the enhanced suppression was due to expression of the cDNA rather than other genetic events such as *lacZ* reporter mutations. To determine if the effect was dependent on the presence of dsRNA, the antisense plasmid was segregated out of the transformants and the strains were again assayed for β-galactosidase activity. Nine of the 25 transformants demonstrated a return to the level of β-galactosidase seen in the parental strain indicating that the effect was dsRNA-dependent (Fig. 9B). The cDNAs being expressed in these transformants were named *R*NAi enhancing sequences (*res*)*.* All transformants displayed normal growth phenotypes indicating that over-expression of the resident cDNAs did not affect general biology of the cell. In the absence of antisense RNA the remaining 16 transformants retained a reduced β-galactosidase activity indicating that the enhanced gene silencing was not dependent on the presence of dsRNA. This suggests that the cDNA-encoded proteins in these strains could have been affecting *lacZ* at the level of transcription or the stability or function of the protein.

The library plasmids were recovered from the aes-containing strains (also referre to as *res*-containing strains) and their cDNA inserts sequenced. BLASTN and BLASTP analysis identified clones W18, W20, and W30 (named *aes2*) as homologues of domains 2 and 3 of the mitochondrial elongation factor Tu (EF Tu). EF Tu is an essential protein which plays a role in transporting tRNA to the A site in the ribosome for peptide elongation. The cDNA in transformants W21, W23, and W32 (named *aes3*) was homologous to a putative protein that was identified in a screen for fission yeast ORFs. Interestingly, the cDNA insert was also homologous to the antisense strand of the 3'UTR of the fission yeast gene sna41 which has previously been shown to be involved in DNA replication. It is thus possible that *aes3* may operate through more than one mechanism in enhancing antisense RNA activity. The cDNA in transformant W47 (named *aes4*) was homologous to the antisense strand of the ribosomal protein L7a, a component of the 60S ribosomal subunit. aes4 also contained a small ORF of unknown biological function. The inserts in transformants W27 and W28 (named *aes1*) shared homology with a putative protein from *C. albicans* that was identified in a screen for genes essential for cell growth. A tertiary quantitative β-galactosidase assay was performed to obtain accurate levels of gene silencing augmentation in the antisense lacZ strains co-expressing these unique factors (Fig. 9C). This analysis showed that these co-factors enhanced antisense suppression by up to 50%. These results demonstrated that over-expression of a cDNA library was an effective way of identifying novel co-factors that magnify the suppressive effect mediated by antisense RNA.

*aes1* shared 43% identity with amino acids 4 to 202 of a *C. albicans* hypothetical protein (AJ390519). *aes2* shared 99% identity with nucleotides 10452 to 9484 of the translation elongation factor EF Tu (AL049769). *aes3* shared 94% identity with nucleotides 776 to 1145 of D89239 *S. pombe* ORF (D89239) and 93% identity with nucleotides 3246 to 2876 of the antisense strand of the DNA replication factor sna41 (AB001739). *aes3* also contained a 220 nt stretch of a GA repeat sequence at its 3' end. *aes4* shared 99% identity with nucleotides 1365 to 584 of the antisense strand of ribosomal protein L7a (AJ001133) and 99% identity with nucleotides 820 to 103 of the antisense strand of ribosomal protein L4 (AB005750). The reference numerals in brackets refer to accession numbers in the GeneBank database (the GeneBank database can be accessed from the following web site: http://www.ncbi.nlm.nih.gov/).

EF Tu is analogous to the eukaryotic EF1α and acts by transporting tRNA to the A site in the ribosome for peptide elongation. EF1α is an essential protein which has also been implicated in a large array of cellular activities including actin binding, microtubule severing, cellular transformation, cell senescence, protein ubiquitination, and protein folding. Detailed analysis of the EF Tu-expressing strain showed that it enhanced antisense RNA-mediated *lacZ* silencing by an additional 15% (Fig. 9C). It has been proposed that dsRNA is fragmented into 21-25 nt species by dsRNA-specific nucleases, amplified by RNA-dependent RNA polymerase, and dissociated by an ATP-dependent RNA helicase. The small antisense fragments are then free to attack homologous mRNA by RNA nuclease-mediated degradation. It has recently been suggested that an associative mechanism could catalyse the ATP-dependent exchange of the sense strand of the short dsRNA with the target mRNA. This may involve the transport of fragmented dsRNA to the ribosome where, in a competitive reaction with tRNA complexes, the complementary antisense strand binds to the mRNA. This would permit the association of the antisense strand of the dsRNA with the target mRNA when the latter is in a structurally exposed state and both inhibit translation and target the mRNA for ribonuclease degradation. Without wishing to be bound by any particular mechanism of action, EF Tu could act by binding to RNAi-dependent short dsRNA species and bring them to the site of action.

The protein encoded by *sna41* has previously been shown to be involved in DNA replication. *sna41* has low homology with *CDC45* and might have DNA helicase properties which could facilitate the expression of complementary sequences. It is conceivable that the antisense plasmid and target DNA sequences may ectopically pair by intermolecular complementarity. Such pairing may inhibit RNA expression and consequently reduce the level of intracellular dsRNA. Similarly the intramolecular pairing of inverted repeat DNA sequences may also interfere with RNA expression. The over-expression of a protein with DNA helicase properties could facilitate the generation of more dsRNA which could in turn enhance the RNAi effect. Furthermore, *CDC45* mutants show an increased rate of plasmid segregation. If plasmid loss is inhibited by over-expression of *sna41* then more dsRNA may be generated leading to more effective RNAi in this system. In this light it is not unreasonable to expect that other proteins normally involved in DNA and/or RNA metabolism and function could also have a role in RNAi modulation and/or enhancement.

The L7a protein is part of the 60s ribosomal sub-unit. Without wishing to be bound by any particular mechanism of action, RNAi augmentation by over-expression of this protein is reasonable as it is hypothesised that the short dsRNA species may undergo strand displacement with target mRNA at the ribosome. The L7a ribosomal protein may act in RNAi by i) mediating docking of dsRNA or its unwound form into the A site of the ribosome, ii) assisting in association of the antisense strand with the target mRNA, and/or iii) shuttling of dsRNA to the ribosomal complex.

Nucleotide sequences representative of the *aes* factors referred to above are provided below and identified as Seq ID Nos 1 to 4 :

### DNA sequence for aes1 factor (Seq ID No 1)

### DNA sequence for aes2 factor (Seq ID No 2)

### DNA sequence for aes3 factor (Seq ID No 3)

### DNA sequence for aes4 factor (Seq ID No 4)

### EXAMPLE 9. Effect of novel RNAi factors on antisense RNA and dsRNA-mediated gene regulation

With recent studies on PTGS suggesting that antisense RNA, co-suppression, and dsRNA-mediated interference may share similar mechanisms, we wanted to determine whether over-expression of an aes factor would also enhance dsRNA-mediated regulation. Having demonstrated that dsRNA could mediate gene suppression in this fission yeast model, the effect of an antisense enhancing sequence on dsRNA-mediated regulation was tested. To this end, the aes2 gene was co-expressed with the lacZ panhandle construct in a yeast strain containing the lacZ target gene. Under these conditions, this transformant displayed an additional 30% suppression of β-galactosidase activity when compared to the transformant expressing only the panhandle lacZ RNA (see Figure 10). This result shows that the aes2 gene, encoding the two domains of the mitochondrial elongation factor EF Tu, stimulated not only antisense RNA- but also dsRNA-mediated gene inhibition.

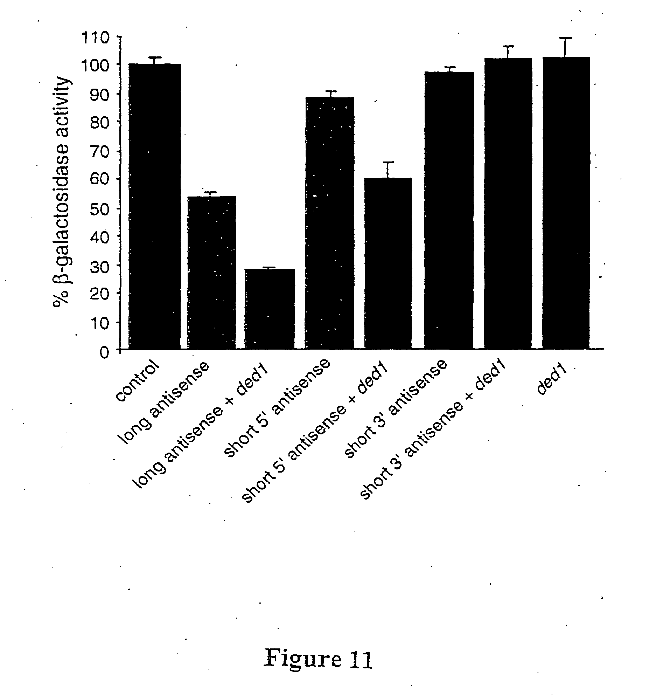

Further it was shown that these two forms of regulation are related by over-expressing the ATP-dependent RNA helicase ded1 in the presence of lacZ antisense RNA and showing that this helicase enhanced gene suppression by a further 50% compared to the control strain (example 6). ded1 was tested on both active and inactive antisense plasmids and demonstrated that ded1 augmentation of gene silencing was dependent on an active antisense RNA (see Figure 11). This could be due to the absence of RNA duplex formation with the inactive antisense RNA and the consequent lack of a substrate for the RNA helicase.

The methods of the present invention have utility in demonstrating a range of RNAi efficacies, in identifying new factors which enhance or reduce gene silencing, in inhibiting gene expression or increasing sensitivity to antisense inhibition of gene expression, in the treatment or prevention of disorders which require inhibition or down-regulation of gene expression.

### REFERENCES

1. Raponi, M., Atkins, D., Dawes, I. & Arndt, G. (2000) Antisense and Nucleic Acid Drug Development 10, 29-34.
2. Arndt, G., Atkins, D., Patrikakis, M. & Izant, J. (1995) Molecular and General Genetics 248,-293-300.
3. Arndt, G., Patrikakis, M. & Atkins, D. (2000) Nucleic Acids Research 28, e15.
4. Clarke, M., Patrikakis, M. & Atkins, D. (2000) Biochemical and Biophysical Research Communications 268, 8-13.
5. Moreno, S. & Nurse, P. (1994) Nature 367, 236-242.
6. Moreno, S., Klar, A. & Nurse, P. (1991) Methods in Enzymology 194, 795-823.
7. Maundrell, K. (1990) Journal of Biological Chemistry 265, 10857-10864.
8. Prentice, H. (1992) Nucleic Acids Research 20, 621.
9. Hoffman, C. & Winston, F. (1987) Gene 57, 267-272.
10. Rose, M., Winston, F. & Hieter, P. (1990) Methods in yeast genetics: A laboratory course manual (Cold Spring Harbor Laboratory Press, Cold Spring Harbor).
11. Maundrell, K. (1993) Gene 123, 127-1390.
12. Basi, G., Schmid, E. & Maundrell, K. (1993) Gene 123, 131-136.
13. Carr, A., MacNeill, S., Hayles, J. & Nurse, P. (1989) Molecular and General Genetics 218, 41-49.
14. Sambrook, J., Fritsch, E. & Maniatis, T. (1989) (Cold Spring Harbor Laboratory - Press, Cold Spring Harbor, New York).
15. Patrikakis, M., Izant, J. & Atkins, D. (1996) Current Genetics 30, 151-158.
16. Brun, C., Dubey, D. & Huberman, J. (1995) Gene 164, 173-177.
17. Heyer, W.-D., Sipiczki, M. & Kohli, J. (1986) Molecular and Cellular Biology 6, 80-89.

## Claims

1. A pharmaceutical composition comprising:
(i) an expressible nucleotide sequence represented by SEQ ID NO 1;
(ii) a nucleic acid encoding a molecule which is, or gives rise to, an anti-sense nucleic acid directed toward at least a portion of the RNA transcript of a target nucleic acid; and
(iii) a pharmaceutically acceptable carrier.

2. A pharmaceutical composition according to claim 1, wherein the nucleotide sequence of (i) and the nucleic acid of ii) are situated on the same or different molecules.

3. A pharmaceutical composition according to claim 1 or 2 wherein the target nucleic acid is an exogenous nucleic acid.

4. An isolated *Schizosaccharomyces pombe* cell having increased susceptibility to anti-sense-mediated inhibition of a target nucleic acid expression, which cell (i) expresses a target nucleic acid and (ii) comprises an elevated level of a nucleotide sequence represented by SEQ ID NO 1, and has a molecule introduced therein which is, or gives rise to, an anti-sense nucleic acid directed toward at least a portion of the RNA transcript of the target nucleic acid, permitting the nucleotide sequence represented by SEQ ID NO 1 to increase the degree to which the anti-sense nucleic acid inhibits expression of the target nucleic acid.

5. A method for inhibiting *ex vivo* the expression of a target nucleic acid in an *Schizosaccharomyces pombe* cell in a cellular preparation, which method comprises the steps of:
(i) increasing or decreasing expression of a nucleotide sequence represented by SEQ ID NO 1 in the cell, and
(ii) prior, concurrently with or subsequent to performing step (i), introducing into the cell a molecule which is, or gives rise to, an anti-sense nucleic acid directed toward at least a portion of an RNA transcript of the target nucleic acid.

6. A method of increasing *ex vivo* cellular susceptibility to anti-sense-mediated inhibition of target nucleic acid expression, which method comprises increasing or decreasing expression of a nucleotide sequence represented by SEQ ID NO 1 in an *Schizosaccharomyces pombe* cell in a cellular preparation that expresses the target nucleic acid, and prior, concurrently or subsequently introducing into the cell a molecule which is, or gives rise to, an anti-sense nucleic acid directed toward at least a portion of the RNA transcript of the target nucleic acid.

7. A method according to claim 5 or 6 wherein the target nucleic acid is an exogenous nucleic acid.

8. A method according to any one of claims 5 to 7 wherein expression of the nucleotide sequence represented by SEQ ID NO. 1 is increased or decreased by introducing into the cell additional copies of, or agents which give rise to, the sequence.

9. An RNAi factor which is a *res* sequence represented by SEQ ID NO. 1.

10. A pharmaceutical composition comprising a *res* sequence represented by SEQ ID NO. 1 together with a pharmaceutically acceptable carrier.

## Patentansprüche

1. Pharmazeutische Zusammensetzung umfassend:
(I) eine exprimierbare Nukleotidsequenz, die dargestellt Ist durch SEQ ID NO.1;
(II) eine Nukleinsäure, die ein Molekül kodiert, das eine anti-sense-Nukleinsäure, die gegen mindestens einen Teil des RNA-Transkripts einer Zielnukleinsäure gerichtet ist, ist oder bildet; und
(III) einen pharmazeutisch akzeptablen Träger.

2. Pharmazeutische Zusammensetzung nach Anspruch 1, wobei die Nukleotidsequenz aus (I) und die Nukleinsäure aus (ii) auf demselben oder verschiedenen Molekülen liegen.

3. Pharmazeutische Zusammensetzung nach Anspruch 1 oder 2, wobei die Zielnukleinsäure eine exogene Nukleinsäure ist.

4. Isolierte S*chizosaccharomyces-pombe*-Zelle, die erhöhte Anfälligkeit für anti-sense-vermittelte Inhibition einer Zielnukleinsäurenexpression aufweist, wobei die Zelle (I) eine Zielnukleinsäure exprimiert und (ii) einen erhöhten Level einer Nukleotidsequenz, die dargestellt ist durch SEQ ID NO. 1, aufweist, und ein darin eingebrachtes Molekül aufweist, das eine anti-sense-Nukleinsäure, die gegen mindestens einen Teil des RNA-Transkripts der Zielnukleinsäure gerichtet ist, ist oder bildet, wobei sie der Nukleotidsequenz, die dargestellt ist durch SEQ ID NO. 1, erlaubt, den Grad, zu dem die anti-sense-Nukleinsäure die Expression der Zielnukleinsäure inhibiert, zu erhöhen.

5. Verfahren zur Inhibition der Expression einer Zielnukleinsäure in einer *Schizosaccheromyces-pombe*-Zelle *ex vivo* in einer Zellpräparation, wobei das Verfahren die Schritte umfasst:
(i) Erhöhten oder Verringern der Expression einer Nukleotidsequenz, die dargestellt ist durch SEQ ID NO. 1, in der Zelle, und
(ii) vor, gleichzeitig oder nach Durchführung des Schritts (i), Einbringen eines Moleküls in die Zelle, das eine anti-sense-Nukleinsäure, die gegen mindestens einen Teil eines RNA-Transkripts der Zielnukleinsäure gerichtet ist, ist oder bildet.

6. Verfahren zum Erhöhen der Zellanfälligkeit für anti-sense-vermittelte Inhibition einer Zielnukleinsäurenexpression *ex vivo*, wobei das Verfahren Erhöhe oder Verringern der Expression einer Nukleotidsequenz, die dargestellt ist durch SEQ ID NO. 1, in einer Schizosaccharomyces-pombe-Zelle in einer Zellpräparation, die die Zielnukleinsäure exprimiert, und vor, gleichzeitig oder nach Einbringen eines Moleküls in die Zelle, das eine anti-sense-Nukleinsäure, die gegen mindestens einen Teil des RNA-Transkripts der Zielnukleinsäure gerichtet ist, ist oder bildet, umfasst.

7. Verfahren nach Anspruch 5 oder 6, wobei die Zielnukleinsäure eine exogene Nukleinsäure ist.

8. Verfahren nach einem der Ansprüche 5 bis 7, wobei die Expression der Nukleotidsequenz, die dargestellt ist durch SEQ ID NO. 1. erhöht oder verringert wird durch Einbringen zusätzlicher Kopien der Sequenz oder von Mitteln, die sie bilden, in die Zelle.

9. RNAi-Faktor, der eine res-Sequenz ist, die dargestellt ist durch SEQ ID NO.1.

10. Pharmazeutische Zusammensetzung umfassend eine res-Sequenz, die dargestellt ist durch SEQ ID NO. 1, zusammen mit einem pharmazeutisch akzeptablen Träger.

## Revendications

1. Composition pharmaceutique comprenant ;
(i) une séquence nucléotidique exprimable représentée par SEQ ID NO:1;
(ii) un acide nucléique codant une molécule qui est, ou donne naissance à, un acide nucléique anti-sens dirigé vers au moins une partie du transcrit d'ARN d'un acide nucléique cible ; et
(iii) un vecteur pharmaceutiquement acceptable.

2. Composition pharmaceutique selon la revendication 1 où la séquence nucléotidique de (i) et l'acide nucléique de (II) sont situés sur la même molécule ou des molécules difFérentes.

3. Composition pharmaceutique selon la revendication 1 ou 2 où l'acide nucléique cible est un acide nucléique exogène.

4. Cellule de *Schizosaccharomyces pombe* Isolée ayant une sensibilité accrue à l'inhibition à médiation anti-sens de l'expression d'un acide nucléique cible, laquelle cellule (i) exprime un acide nucléique cible et (ii) comprend un niveau élevé d'une séquence nucléotidique représentée par SEQ ID NO:1, et a une molécule introduite dans celle-ci qui est, ou donne naissance à, un acide nucléique anti-sens dirigé vers au moins une partie du transcrit d'ARN de l'acide nucléique cible, en permettant à la séquence nucléotidique représentée par SEQ ID NO:1 d'augmenter le degré auquel l'acide nucléique anti-sens inhibe l'expression de l'acide nucléique cible.

5. Procédé pour inhiber *ex vivo* l'expression d'un acide nucléique cible dans une cellule de *Schizosaccharomyces pombe* dans une préparation cellulaire, lequel procédé comprend les étapes de :
(i) augmentation ou diminution de l'expression d'une séquence nucléotidique représentée par SEQ ID NO:1 dans la cellule, et
(ii) avant, pendant ou après l'accomplissement de l'étape (i), introduction dans la cellule d'une molécule qui est, ou donne naissance à, un acide nucléique anti-sens dirigé vers au moins une partie d'un transcrit d'ARN de l'acide nucléique cible.

6. Procédé d'augmentation *ex vivo* de la sensibilité cellulaire à l'inhibition à médiation anti-sens de l'expression d'un acide nucléique cible, lequel procédé comprend l'augmentation ou la diminution de l'expression d'une séquence nucléotidique représentée par SEQ ID NO:1 dans une cellule de *Schizosaccharomyces pombe* dans une préparation cellulaire qui exprime l'acide nucléique cible, et avant, pendant ou après l'introduction dans la cellule d'une molécule qui est, ou donne naissance à, un acide nucléique anti-sens dirigé vers au moins une partie du transcrit d'ARN de l'acide nucléique cible.

7. Procédé selon la revendication 5 ou 6 où l'acide nucléique cible est un acide nucléique exogène.

8. Procédé selon l'une quelconque des revendications 5 à 7 où l'expression de la séquence nucléotidique représentée par SEQ ID NO:1 est augmentée ou diminuée par introduction dans la cellule de copies supplémentaires de, ou d'agents qui donnent naissance à, la séquence.

9. Facteur ARNi qui est une séquence *res* représentée par SEQ ID NO:1.

10. Composition pharmaceutique comprenant une séquence res représentée par SEQ ID NO:1 en même temps qu'un vecteur pharmaceutiquement acceptable.
